# EUROPEAN PATENT APPLICATION

(11) **EP 3 332 794 A1**
(43) Date of publication of application: **13.06.2018**
(21) Application number: 16833090.0
(22) Date of filing: 03.08.2016
(51) Int. Cl.: A61K 36/63, A23L 33/10, A61K 9/107, A61K 9/72, A61K 36/28, A61K 36/286, A61K 36/31, A61K 36/534, A61K 47/10, A61K 47/14, A61P 11/00

(54) **EMULSION COMPOSITION FOR PREVENTING OR AMELIORATING SNORING**

(30) Priority: 04.08.2015 JP 2015154194
(71) Applicant: Nitto Pharmaceutical Industries, Ltd., Muko-shi, Kyoto 617-0006 (JP)
(72) Inventor: KITAO, Tetsurou, Muko-shi Kyoto 617-0006 (JP); MORI, Yoshiro, Muko-shi Kyoto 617-0006 (JP); YAMADA, Koichi, Muko-shi Kyoto 617-0006 (JP); TANAKA, Miki, Muko-shi Kyoto 617-0006 (JP)
(74) Representative: J A Kemp
(86) International application number: PCT/JP2016/072867
(87) International publication number: WO 2017/022811

(57) **Abstract**

The present invention provides an emulsion composition containing (A) a vegetable oil, (B) a sugar alcohol having a carbon number of not less than 4 and (C) an emulsifier, or further, (D) a plant essential oil, which composition is superior in emulsion stability, suppresses separation and the like of the contained components during preservation, can be applied conveniently to a pharyngeal region without requiring a homogenizing operation at the time of application, and can be ingested or used continuously.

## Description

### [Technical Field]

The present invention relates to an emulsion composition for preventing or improving snoring. More particularly, the present invention relates to an emulsion composition for preventing or improving snoring showing superior emulsion stability, permitting convenient application to a pharyngeal region, and enabling continuous ingestion or use.

### [Background Art]

"Snoring" is a sound produced by vibration, on entry and exit of the air during breathing, of the soft palate and tongue base blocking the upper respiratory tract to narrow same due to various diseases and organic abnormalities, disorders, temporary fatigue or drinking and the like. It also refers to a pathology in which such sound continues day after day.

The causes of narrowing of the upper respiratory tract include (1) obesity, (2) large uvula, (3) swelling due to tonsils and adenoid inflammation etc., (4) large tongue or large soft palate, (5) diseases accompanying nasal obstruction such as allergic rhinitis, nasal polyp, empyema, etc., (6) muscle weakness of the tongue due to influences of aging, fatigue, drinking, drugs such as sleeping agent, etc., and the like.

Snoring has a great influence not only on health, but also on social and interpersonal aspects. With respect to health, deterioration of sleep quality, even when it is not a disease such as sleep apnea syndrome or sleep hypopnea, sometimes interferes with daytime activities. Also, with respect to the social and interpersonal aspects, the sense of shame may lead to the avoidance of group travel, and snoring sometimes disturbs sleeping of people living together.

Clips that are inserted into the nostrils and sandwich the nasal septum and mouthpieces are sold as medical devices for preventing or improving snoring. CPAP (Continuous Positive Airway Pressure) as a treatment apparatus for sleep apnea syndrome is also said to have the effect of extinguishing snoring.

To improve nasal obstruction, nose drops containing a vasoconstrictor such as naphazoline nitrate, tetrahydrozoline hydrochloride and the like are used. Furthermore, to moisten nasal mucosa, nose drops containing glycerol are used, and compositions containing any one kind or two or more kinds of eucalyptus oil, peppermint oil, peppermint oil, menthol and cineole, utilization of sansonin-to and the like have also been proposed (patent documents 1, 2).

However, clips for nose and mouthpieces sometimes cause foreign body sensation or an uncomfortable feeling when in use, and it is difficult to say that treatment apparatuses such as CPAP and the like can be utilized conveniently. In addition, nasal drops containing vasoconstrictor have been reported to cause side effects such as hypersensitivity, nervousness, elevation of blood pressure, irritative pain, feeling of dryness, nausea, cardiac palpitation and the like, and consecutive use for a long term may cause thickened nasal mucosa. With respect to nasal drops aiming at moistening of the nasal mucosa and the like, use of a wetting agent at a concentration capable of obtaining a sufficient wetting effect of the nasal mucosa may cause inflammation by giving irritation to the nasal mucosa. Furthermore, snoring often persists chronically; however, pharmaceutical products including traditional Chinese medicine are not suitable for continuous ingestion or use.

In view of the above-mentioned problem, the present inventors have proposed and already disclosed a composition for preventing or improving snoring containing olive oil, sunflower oil and peppermint oil as a composition that can be conveniently applied to a pharyngeal region without causing foreign body sensation or an uncomfortable feeling, is effective for preventing or improving snoring, is also superior in safety, and can be ingested or used continuously (patent document 3).

In consideration of convenient application to a pharyngeal region, texture at the pharyngeal region when applied, a preventive or improving effect on snoring and the like, the above-mentioned composition is preferably prepared as an emulsion composition comprising an oily component containing olive oil, sunflower oil and peppermint oil emulsified and dispersed in a polar solvent such as water and the like. However, it was newly found that an emulsion composition prepared in such manner sometimes shows poor emulsion stability and causes problems such as separation of oily component during preservation and the like. When separation of oily component and the like occurs during preservation, a homogenizing operation such as shaking and mixing immediately before application to a pharyngeal region becomes necessary and when the composition is used without the homogenizing operation, the effect of the composition may not be obtained sufficiently. Thus, there is a concern that the composition may become a product that cannot be used conveniently by the user.

### [Document List]

### [Patent documents]

patent document 1: JP-A-8-333244
patent document 2: JP-A-2009-13173
patent document 3: JP-A-2013-121929

### [SUMMARY OF THE INVENTION]

### [Problems to be Solved by the Invention]

Accordingly, the present invention aims to provide an emulsion composition for preventing or improving snoring, that shows good emulsion stability, suppresses separation and the like of the contained components such as oily component and the like during preservation, does not require homogenizing operation at the time of application, can be applied conveniently to a pharyngeal region, and further, can be continuously ingested or used.

### [Means of Solving the Problems]

The present inventors have conducted intensive studies in an attempt to solve the above-mentioned problems and found that an emulsion composition containing (A) a vegetable oil, (B) a sugar alcohol having a carbon number of not less than 4 and (C) an emulsifier is superior in emulsion stability. They have found that separation and the like of the contained components such as oily component and the like is suppressed during preservation in the aforementioned composition, and therefore, the composition does not require a homogenizing operation at the time of application and can be applied to a pharyngeal region conveniently orally or by application to the oral cavity without causing a foreign body sensation or an uncomfortable feeling, and further, that the aforementioned composition satisfactorily moistens and softens the mucosa of the pharyngeal region and can better prevent or improve snoring, which resulted in the completion of the present invention.

Therefore, the present invention provides the following.
[1] An emulsion composition for preventing or improving snoring, comprising (A) a vegetable oil, (B) a sugar alcohol having a carbon number of not less than 4 and (C) an emulsifier.
[2] The emulsion composition of the above-mentioned [1], further comprising (D) a plant essential oil.
[3] The emulsion composition of the above-mentioned [1] or [2], wherein the (A) vegetable oil is one kind or two or more kinds selected from the group consisting of canola oil, olive oil, sunflower oil and safflower oil.
[4] The emulsion composition of any of the above-mentioned [1] - [3], wherein the (B) sugar alcohol having a carbon number of not less than 4 is one kind or two or more kinds selected from alditols having a carbon number of 5 - 6.
[5] The emulsion composition of any of the above-mentioned [1] - [4], wherein the (C) emulsifier is one kind or two or more kinds selected from the group consisting of glycerin fatty acid ester, sorbitan fatty acid ester and organic acid monoglyceride.
[6] The emulsion composition of any of the above-mentioned [2] - [5], wherein the (D) plant essential oil is an essential oil obtained from one kind or two or more kinds of plants of the genus Mentha.
[7] The emulsion composition of any of the above-mentioned [1] - [6], which is a liquid.
[8] The emulsion composition of any of the above-mentioned [1] - [7], which is sprayed on the pharynges.
[9] An agent for preventing or improving snoring, comprising the emulsion composition of any of the above-mentioned [1] - [6].
[10] The agent of the above-mentioned [9], which is an oral liquid.
[11] The agent of the above-mentioned [9], which is a spray for pharynges.
[12] A food for preventing or improving snoring, comprising the emulsion composition of any of the above-mentioned [1] - [6].
[13] The food of the above-mentioned [12], which is a liquid.
[14] The food of the above-mentioned [12], having a form of a spray.
[15] The food of any of the above-mentioned [12] - [14], which is a food with health claims, special purpose food or health supplement.

### [Effect of the Invention]

The emulsion composition for preventing or improving snoring of the present invention is superior in emulsion stability and better suppresses separation and the like of the contained components such as oily component and the like during preservation.

Therefore, the emulsion composition for preventing or improving snoring of the present invention can be applied conveniently to a pharyngeal region without requiring a homogenizing operation at the time of application and without causing a foreign body sensation or an uncomfortable feeling, and can prevent or improve snoring effectively.

Furthermore, the emulsion composition for preventing or improving snoring of the present invention is hypoallergenic and highly safety, and is suitable for continuous ingestion or use for a long term.

### [Brief Description of the Drawings]

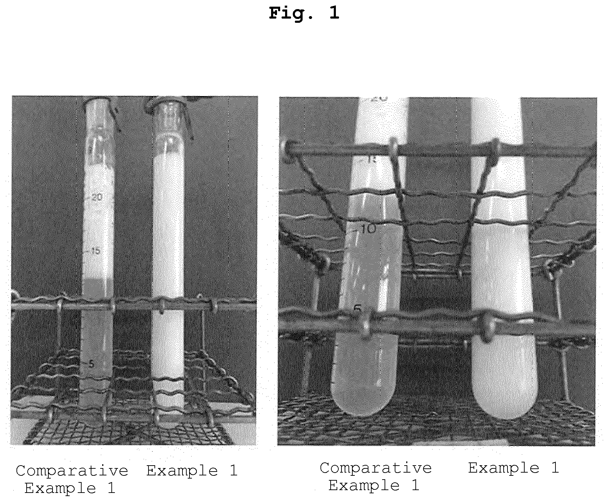

Fig. 1 shows the state of the respective emulsion compositions of Example 1 and Comparative Example 1 of the present invention, which were placed in test tubes with a stopper, sufficiently shaken after setting the stopper and allowed to stand at room temperature for 4 weeks.

### [Description of Embodiments]

The emulsion composition for preventing or improving snoring of the present invention (hereinafter to be also referred to as "the emulsion composition of the present invention") contains (A) a vegetable oil, (B) a sugar alcohol having a carbon number of not less than 4 and (C) an emulsifier.

Also, the emulsion composition of the present invention may further contain (D) a plant essential oil.

In the present invention, as the (A) vegetable oil, any oil can be used without particular limitation as long as it is extracted and purified from a plant and can be applied to a pharyngeal region. An oil suitable as a carrier or oil agent for oral preparation or suitable for food is preferably used.

As the (A) vegetable oil, avocado oil, flaxseed oil, almond oil, apricot oil, perilla oil, olive oil, black currant oil, cashew oil, canola oil, walnut oil, sesame oil, wheat oil, rice oil, Japanese basil oil, soybean oil, evening primrose oil, camellia oil, corn oil, rape seed oil, sunflower oil, grapes seed oil, hazelnut oil, safflower oil, macadamia oil, cottonseed oil, coconut oil, peanuts oil and the like can be mentioned. One kind of these can be used alone, or two or more kinds thereof may be used in combination.

In the present invention, as the (A) vegetable oil, a crude oil obtained by, according to a method known per se, compressing flower, leaves, seeds, fruits and the like of various plants or extracting same with a solvent and purifying same can also be used. It is convenient to use a product commercially available from each company as an oil for oral preparation or food.

From the aspect of a preventing or improving effect on snoring by the emulsion composition of the present invention, vegetable oil, for example, canola oil, olive oil, sunflower oil, safflower oil and the like, rich in oleic acid and having a less content of saturated fatty acid is preferably used as a constituent fatty acid of fats and oils. In the emulsion composition of the present invention, one kind or two or more kinds selected from the group consisting of the aforementioned vegetable oils are preferably used as the (A) vegetable oil, and olive oil and sunflower oil are particularly preferably used.

As olive oil particularly preferably used in the emulsion composition of the present invention is a vegetable oil obtained from olive (Olea europaea) fruit, and the olive oil (Oleum Olivae) defined in the Japanese Pharmacopoeia, or an oil having properties and the like suitable as food and the like, is preferably used for the object of the present invention.

Sunflower oil is fat or oil obtained from sunflower (Helianthus annuus) seeds, or sunflower oil having properties and the like suitable as food and the like, is preferably used for the object of the present invention.

The content of the (A) vegetable oil in the emulsion composition of the present invention is preferably 2.5 wt% - 50 wt%, more preferably 5 wt% - 25 wt%, relative to the total amount of the composition.

In the present invention, as the (B) sugar alcohol having a carbon number of not less than 4, alcohol produced by reduction of an aldehyde group of aldose having a carbon number of not less than 4, or a carbonyl group of ketose having a carbon number of not less than 4, and applicable to a pharyngeal region can be used without particular limitation. An alcohol suitable for oral preparation or food is preferable.

As the (B) sugar alcohol having a carbon number of not less than 4, monosaccharide sugar alcohol, for example, tetritol such as erythritol, D-threitol and the like; pentitol such as D-arabinitol, xylitol, ribitol, xylulitol, ribulitol and the like; hexitol such as galactitol, sorbitol, mannitol, fructitol and the like; heptitol such as volemitol, perseitol and the like; octitol such as D-erythro-D-galacto-octitol and the like, disaccharide sugar alcohol, for example, lactitol, maltitol and the like can be mentioned. One kind of these can be used alone, or two or more kinds thereof may be used in combination. In addition, a mixture of sugar alcohol such as HSH (hydrogenated starch hydrolysates), isomalto and the like can also be used.

The above-mentioned sugar alcohol can also be extracted, purified from a plant etc. or synthesized according to a method known per se and used. However, it is convenient to use a product provided by each company as sugar alcohol for oral preparation or food.

From the aspect of the emulsion stability of the emulsion composition of the present invention, alditol having a carbon number of not less than 4 (sugar alcohol produced by reduction of aldehyde group of aldose) is preferable as the (B) sugar alcohol having a carbon number of not less than 4. Of these, pentitol such as D-arabinitol, xylitol, ribitol and the like and hexitol such as galactitol, sorbitol, mannitol and the like are more preferable, and pentitol is still more preferable. Particularly, xylitol is preferably used.

The content of the (B) sugar alcohol having a carbon number of not less than 4 in the emulsion composition of the present invention is preferably 10 wt% - 60 wt%, more preferably 20 wt% - 30 wt%, of the total amount of the emulsion composition.

In the present invention, as the (C) emulsifier, an amphiphilic substance capable of satisfactorily emulsifying and dispersing the above-mentioned (A) vegetable oil, and further, the below-mentioned (D) plant essential oil, in an aqueous phase containing the above-mentioned (B) sugar alcohol having a carbon number of not less than 4, and applicable to a pharyngeal region can be used without particular limitation. A non-ionic emulsifier is preferably used, and an emulsifier for an oral preparation or food is more preferable.

As the (C) emulsifier, lecithin such as enzyme-treated lecithin, enzyme-degraded lecithin, plant lecithin, fractionated lecithin, egg-yolk lecithin and the like; glycerin fatty acid ester such as glycerol monopalmitate, glycerol monostearate, glycerol monooleate and the like; sucrose fatty acid ester such as sucrose palmitate, sucrose stearate, sucrose oleate and the like; sorbitan fatty acid ester such as sorbitan monolaurate, sorbitan monopalmitate, sorbitan monostearate, sorbitan monooleate and the like; organic acid monoglyceride such as acetic acid monoglyceride, lactic acid monoglyceride, citric acid monoglyceride, diacetyltartaric acid monoglyceride and the like; polyoxyethylene sorbitan fatty acid ester such as polyoxyethylene(20)sorbitan monooleate, polyoxyethylene(20)sorbitan monostearate, polyoxyethylene(20)sorbitan tristearate, polyoxyethylene(20)sorbitan monolaurate and the like; polyglycerol fatty acid ester such as diglycerol monooleate, decaglycerol monolaurate, decaglycerol monomyristate, decaglycerol monostearate, decaglycerol monoisostearate and the like; polyglycerol condensed ricinoleic acid ester such as hexaglycerol polyricinoleate, decaglycerol polyricinoleate and the like; propylene glycol fatty acid ester such as propylene glycol monostearate and the like; and the like can be mentioned. One kind of these can be used alone, or two or more kinds thereof may be used in combination.

The above-mentioned emulsifier may be synthesized according to a method known per se and used. However, it is convenient to use a product provided by each company as an emulsifier for oral preparation or food.

From the aspect of emulsion stability of the emulsion composition, glycerin fatty acid ester, sorbitan fatty acid ester or organic acid monoglyceride is preferably used as the (C) emulsifier, organic acid monoglyceride is more preferable, and citric acid monoglyceride is particularly preferable.

The content of the (C) emulsifier in the emulsion composition of the present invention is preferably 1 wt% - 10 wt%, more preferably 2 wt% - 5 wt%, of the total amount of the emulsion composition.

The (D) plant essential oil to be used in the present invention is a volatile oil having a specific fragrance produced by the plant. Any plant essential oil can be used in the emulsion composition of the present invention without particular limitation as long as it is applicable to a pharyngeal region, and one suitable for oral preparation or food is preferably used.

As the (D) plant essential oil, essential oil harvested from flower and flower bud of rose, jasmine, camomile, ylang-ylang and the like; leaf of geranium, eucalyptus, tea tree and the like; fruit skin of citrus (Citrus unshiu, seville orange, orange, lemon, lime, bergamot etc.); fruit and seed of anise, allspice, clove, pepper, Japanese pepper, ginger, nutmeg, juniper, vanilla and the like; tree bark of sandalwood, pine, Japanese cypress, cinnamon and the like; root and rootstock of vetiver and the like; leaf and whole plant of a plant of the genus Mentha (lavender, lemongrass, basil, rosemary, mint etc.) and the like, and the like can be mentioned. One kind of these can be used alone, or two or more kinds thereof may be used in combination.

The above-mentioned plant essential oil may be collected from respective parts of various plants by a distillation method such as steam distillation method, high temperature carbonization method, fractional distillation method, low temperature vacuum extraction method and the like; enfleurage such as cold extraction method, warm extraction method and the like; an extraction method such as solvent extraction method, supercritical fluid extraction method and the like; a compression method and the like and used. However, it is convenient to use a product provided by each company as an oil for oral preparation or food.

From the aspect of a preventing or improving effect on snoring by the emulsion composition of the present invention, an essential oil obtained from one kind or two or more kinds of the plant of the genus Mentha is preferably used as the (D) plant essential oil.

As the above-mentioned plant of the genus Mentha, Mentha aquatica, Mentha arvensis, Mentha canadensis L. var. piperascens, Mentha citrata, Mentha haplocalyx, Mentha japonica, Mentha pulegium, Mentha spicata, Mentha suaveolens, Mentha x piperita L. and the like can be mentioned. An essential oil collected from these leaves or whole plant by the above-mentioned method is used.

Of these, an essential oil containing a large amount of menthol such as peppermint oil obtained from Mentha canadensis L. var. piperascens, peppermint oil obtained from Mentha x piperita L. and the like is more preferably used, and one having a menthol content of not less than 30 wt% such as peppermint oil (Oleum Menthae Japonicae) defined in the Japanese Pharmacopoeia, "peppermint oil" and the like provided by Frey+Lau and the like are particularly preferably used.

The content of the (D) plant essential oil in the emulsion composition of the present invention is preferably 0.5 wt% - 10 wt%, more preferably 1 wt% - 5 wt%, relative to the total amount of the emulsion composition.

In the emulsion composition of the present invention, from the aspect of emulsion stability, the (A) vegetable oil, (B) sugar alcohol having a carbon number of not less than 4 and (C) emulsifier are preferably contained at 3 - 9:7 - 27:1 in a weight ratio of these [(A):(B):(C)].

From the aspect of imparting humidity to the mucosa of the pharyngeal region, imparting an anti-inflammatory effect and the like, the emulsion composition of the present invention preferably further contains vitamins.

As the vitamin, vitamin A, vitamin B group, vitamin C and vitamin E can be used.

As vitamin A, retinol; retinol derivative such as retinoic acid, retinol acetate, retinol palmitate and the like; vitamin A oil which is a mixture of these and the like can be mentioned.

As the vitamin B group, vitamin B₁ and a derivative thereof such as thiamine, thiamine hydrochloride, thiamine pyrophosphate and the like; riboflavin, vitamin B₂ and a derivative thereof such as riboflavin butyrate and the like; niacin such as nicotinic acid, nicotinic acid amide and the like; pantothenic acid and a derivative thereof such as pantothenic acid, pantothenyl alcohol, pantethine and the like; vitamin B₆ and a derivative thereof such as pyridoxine hydrochloride, pyridoxal, pyridoxal phosphate, pyridoxamine and the like; biotin; folic acid and a derivative thereof such as folic acid, dihydrofolic acid, folinic acid and the like; vitamin B₁₂ such as cyanocobalamin, hydroxocobalamin, methylcobalamin and the like, and the like can be mentioned.

As vitamin C, ascorbic acid, and ascorbic acid derivatives such as dehydroascorbic acid, ascorbyl palmitate and the like can be mentioned.

As vitamin E, natural vitamin E such as D-α-tocopherol, D-β-tocopherol, D-γ-tocopherol, D-δ-tocopherol, D-α-tocopherol acetate, tocotrienol and the like; synthetic vitamin E such as DL-α-tocopherol, DL-α-tocopherol acetate and the like and the like can be mentioned.

In the present invention, one kind of the above-mentioned vitamin can be used alone, or two or more kinds thereof may be used in combination. From the aspect of the effect of imparting humidity to the mucosa of the pharyngeal region, and the like, one kind or two or more kinds selected from vitamin B group, vitamin C and vitamin E is preferably used, and one or more kinds selected from each group of vitamin B group, vitamin C and vitamin E is more preferably used. As a vitamin selected from vitamin B group, vitamin B₆ is still more preferably used.

The content of the above-mentioned vitamin in the emulsion composition of the present invention is preferably 0.1 wt% - 5.0 wt%, more preferably 0.5 wt% - 1.0 wt%.

An antioxidant, preservative, pH adjuster, flavor, corrigent, colorant and the like can be further added to the emulsion composition of the present invention as long as the characteristics of the present invention are not impaired.

As the antioxidant, erythorbic acid, sodium erythorbate, γ-oryzanol, quercetin, dibutylhydroxytoluene, sage extract, butylhydroxyanisole, gallic acid, rutin and the like can be mentioned.

As the preservative, sodium benzoate, disodium ethylenediaminetetraacetate, sorbic acid, potassium sorbate, sodium dehydroacetate, ethyl paraoxybenzoate, propyl paraoxybenzoate, butyl paraoxybenzoate, sodium pyrosulfite, ε-polylysine and the like can be mentioned.

As the pH adjuster, hydrochloric acid, sulfuric acid, sodium sulfate, phosphoric acid, potassium dihydrogen phosphate, sodium dihydrogen phosphate, trisodium phosphate, citric acid, trisodium citrate, lactic acid, sodium lactate, potassium hydroxide, sodium hydroxide and the like can be mentioned.

As the flavor, acetophenone, isoeugenol, eugenol, geraniol, cinnamic acid, citronellol, 1,8-cineol, cinnamaldehyde, vanillin, D-borneol, linalool, lemon oil and the like can be mentioned.

As the corrigent, sweetening agent such as aspartame, acesulfame potassium, licorice extract, saccharin sodium, sucrose, sucralose, steviaan extract and the like; souring agent such as adipic acid, succinic acid, acetic acid, L-tartaric acid, L-sodium tartarate, phytic acid, DL-malic acid and the like, bittering agent such as caffeine, Gentiana lutea extract and the like, and the like can be mentioned.

As the colorant, inorganic pigment such as red ferric oxide, titanium oxide and the like; natural dye such as annatto dye, turmeric dye, β-carotene, gardenia yellow dye, chlorophyll, safflower red dye, safflower yellow dye, strawberry dye, tamarind dye, grapes fruit skin dye, blueberry dye and the like; tar pigment such as Food Color Yellow No. 4, Food Color Yellow No. 4 aluminum lake, Food Color Blue No. 1, Food Color Blue No. 1 aluminum lake, Food Color Blue No. 2, Food Color Blue No. 2 aluminum lake, Food Color Red No. 102, Food Color Red No. 104, Food Color Red No. 2, Food Color Red No. 2 aluminum lake, Food Color Green No. 3, Food Color Green No. 3 aluminum lake and the like, and the like can be mentioned.

The emulsion composition of the present invention can be produced according to a general production method of an emulsion composition for a pharmaceutical product or food. For example, (A) a vegetable oil and (C) an emulsifier are mixed and other oil-soluble components are added as necessary and mixed to give an oil phase component, which is heated to 75°C - 80°C. On the other hand, (B) a sugar alcohol having a carbon number of not less than 4 is dissolved in a solvent used generally, other water-soluble components are added as necessary and mixed to give an aqueous phase component, which is heated to 75°C - 80°C. The aforementioned oil phase component was added to and mixed with the aforementioned aqueous phase component under stirring to uniformly emulsify them. Then the mixture is cooled and, when cooled to about 40°C, (D) a plant essential oil, vitamin, flavor, pH adjuster and the like is added where necessary and mixed, whereby the emulsion composition can be prepared.

As a solvent to dissolve (B) a sugar alcohol having a carbon number of not less than 4, polar solvents such as water; lower alcohol (e.g., ethanol and the like); polyhydric alcohol (e.g., propanediol, butanediol, glycerol and the like) and the like are preferably used from the aspects of a moistening effect on a pharyngeal region, handling property of the emulsion composition and the like, and water and glycerol are more preferable from the aspects of a moistening effect on a pharyngeal region and hypoallergenic property.

In the present invention, a homomixer, a colloid mill, a high-pressure homogenizer and the like can be used for homogenization to achieve emulsification.

The emulsion composition for preventing or improving snoring of the present invention is preferably provided in a form directly applicable to a pharyngeal region, such as oral application or application to oral cavity and the like, and can be provided in the form of a liquid, semi-solid, aerosol and the like.

The aerosol emulsion composition can be prepared by filling the above-mentioned liquid emulsion composition in a container together with a liquefied gas or compressed gas or in a container with a pump dispenser to be in a sprayable form.

For the object of the present invention, a liquid emulsion composition is preferably in a form for spraying in a mist.

It is preferable to ingest or use 50 mg - 600 mg, more preferably 100 mg - 300 mg, as the (A) vegetable oil, or the total amount of (A) vegetable oil and (D) plant essential oil of the emulsion composition for preventing or improving snoring of the present invention per day for an adult. The aforementioned amount of the emulsion composition of the present invention may be ingested or used as one dose or in several divided portions.

The emulsion composition of the present invention is superior in emulsion stability, well suppresses separation and the like of the contained components during preservation, and therefore, can be applied conveniently to a pharyngeal region without requiring a homogenizing operation at the time of application.

The emulsion composition of the present invention shows a mild action and contains a hypoallergenic vegetable oil, further a plant essential oil, as an active ingredient, and therefore, it shows low stimulation to a pharyngeal region and oral mucosa and also high safety and can be ingested or used continuously.

The above-mentioned emulsion composition for preventing or improving snoring of the present invention can be used as it is, or contained in pharmaceutically acceptable carrier to give an agent for preventing or improving snoring. As the pharmaceutically acceptable carrier, liquid carriers such as water, lower alcohol such as ethanol and the like, polyhydric alcohol such as glycerol and the like, and a mixed solution of these and the like, powder carriers such as emulsion, gel, excipient powder and the like, and the like can be mentioned.

Therefore, the agent for preventing or improving snoring of the present invention can be provided as an oral liquid such as elixir, suspension, emulsion, lemonade and the like; syrup; oral jelly agent; oral cavity tablet such as troche, gum agent and the like; tablet such as orally disintegrating tablet, chewable tablet and the like; spray such as oral cavity spray, pharynges spray and the like; oral rinse; mouth rinse and the like.

An agent for preventing or improving snoring in the above-mentioned dosage form can be produced using the emulsion composition for preventing or improving snoring of the present invention, and adding, as necessary, additives generally used for formulating such as emulsifier or suspending or dispersing agent, thickening or gelling agent, excipient, binder, disintegrant, lubricant, antioxidant, preservative, pH adjuster, flavor, corrigent, colorant and the like, according to a general production method such as the production method described in the Sixteenth revised Japanese Pharmacopoeia Preparation General Rules [2] each item of Preparation, or Seventeenth revised Japanese Pharmacopoeia Preparation General Rules [3] each item of Preparation and the like.

In consideration of the easiness and efficiency of the application to a pharyngeal region, and sustainability of a moistening effect at a pharyngeal region and the like, the agent for preventing or improving snoring of the present invention is preferably formulated as an oral liquid or a spray directly applicable to a pharyngeal region, more preferably a spray for pharynx.

When the agent for preventing or improving snoring of the present invention is formulated as a spray for pharynx, the above-mentioned liquid emulsion composition of the present invention can be filled in a general spray container to produce same.

While the spray container to be used in the present invention is not particularly limited as long as it is suitable for spray application to a pharyngeal region, a wide mouth container with a pump dispenser having a dip tube attached thereto is preferably used. To control the application amount of the agent for preventing or improving snoring of the present invention, a spray container having a metered-dose ejection mechanism capable of controlling the ejection amount by one press of the pump dispenser to a certain amount is preferably used. As such metered-dose ejection mechanism, a metered-dose ejection mechanism generally equipped to a spray container used in the field of otorhinolaryngology can be used.

When the above-mentioned emulsion composition of the present invention is filled in a spray container with a metered-dose ejection mechanism to provide as a spray for pharynx, the ejection amount by one press of the pump is preferably set to 0.1 mL - 4.0 mL, more preferably 0.2 mL - 2.0 mL. The spray for pharynx of the present invention is preferably used by pressing the pump one to several times, more preferably about 3 times, for one application to an adult. The number of applications per day varies depending on the age of the user, level of snoring and the like. In consideration of the above-mentioned amount of ingestion or use of the composition of the present invention per day for an adult, it is generally about 1 - 6 times, preferably about 1 - 4 times, more preferably about 1 - 3 times. The spray for pharynx of the present invention is more effectively used before going to bed.

In the present invention, a preparation component filling part of a spray container is generally a bottle made of glass or plastic resin. To prevent degradation of the preparation components due to light, a shading container by colored glass and the like is preferably used. When plastic resin is used, one impermeable to vegetable oil and plant essential oil is preferably used. In addition, a pump dispenser made of plastic resin is generally used.

As the above-mentioned pump dispenser and spray container with metered-dose ejection mechanism to be used for the spray for pharynx of the present invention, one commercially available for a spray in the otorhinolaryngology field can be used.

The spray for pharynx of the present invention can be preferably produced by filling the above-mentioned liquid emulsion composition of the present invention in a preparation component filling part of a spray container, performing a sterilization treatment and setting a pump dispenser.

The agent for preventing or improving snoring of the present invention is ingested or used such that the above-mentioned amount of the emulsion composition of the present invention can be ingested or used per day for an adult. That is, the agent is ingested or used such that preferably 50 mg - 600 mg, more preferably 100 mg - 300 mg, of the (A) vegetable oil, or the total amount of (A) vegetable oil and (D) plant essential oil can be ingested or used per day.

The emulsion composition for preventing or improving snoring of the present invention can be used as it is, or contained in a food carrier and added with a food additive as necessary to give a food for preventing or improving snoring. As used herein, the "food" refers to a "food" defined in Article 4 of the Food Sanitation Act, namely, food and drink. The food for preventing or improving snoring of the present invention can be provided in various forms such as liquid, semi-solid, solid and the like.

As the liquid food, soup, sauce, syrup, dressing, beverage and the like can be mentioned. As the beverage, fruit drinks such as fruit juice beverage, fruit flesh beverage and the like; vegetable drinks such as tomato juice, carrot juice, vegetable juice and the like; cow milk; soymilk; lactobacillus drinks; coffee; cocoa drinks; energy drink and the like can be mentioned.

As the semi-solid food, jelly, jam, yogurt, cream and the like can be mentioned.

As the solid food, confectionery such as drop, candy, lozenge, chewing gum, *ramune* candy, tablet confectionery such as mint tablet and the like, and the like; powder drinks such as powder juice, instant coffee, instant cocoa and the like; powder food such as instant soup and the like; gummi; capsule food and the like can be mentioned.

In the present invention, provision in the form of liquid food such as beverage and the like, candy confectionery such as lozenge and the like, orally disintegratable tablet confectionery or capsule food is preferable from the aspects of easy ingestion, sustainability of moistening effect on pharyngeal region and the like. The liquid food can also be provided in a spray form.

The food for preventing or improving snoring of the present invention can be produced using the above-mentioned emulsion composition for preventing or improving snoring of the present invention, and adding, as necessary, food additives such as sweetener, colorant, preservatives, thickening stabilizer, gelation agent, antioxidant, coloring agent, bleaching agent, fungicide, emulsifier, expansion agent, seasoning, acidulant, bittering agent, gloss agent, gum base, nutrition enhancer, manufacturing agent, fragrance and the like, according to a general production method of food. In addition, a food in a spray form can be produced according to the above-mentioned production method of a spray for pharynx.

The food for preventing or improving snoring of the present invention can also be provided in the form of particularly spray, energy drink, tablet confectionery, capsule and the like, and as food with health claims such as food for specified health uses, food with nutrient function claims, indicated functional food and the like; special purpose foods such as food for sick people, food for the elderly and the like; health supplement and the like.

The ingestion amount of the food for preventing or improving snoring of the present invention is preferably 50 mg - 600 mg, more preferably 100 mg - 300 mg, of the (A) vegetable oil, or the total amount of (A) vegetable oil and (D) plant essential oil per day for an adult. In the present invention, the aforementioned ingestion amount may be ingested as one dose or in several divided portions.

When the food for preventing or improving snoring of the present invention is provided as the above-mentioned food with health claims, special purpose food, health supplement and the like, an ingestion amount per dose of the above-mentioned food for preventing or improving snoring of the present invention is preferably contained in food in a form packed or filled in the unit of one meal intake. The "food in a form packed or filled in the unit of one meal intake" means that an amount of food to be ingested at one time is packed or filled in a container such as one bag, box, bottle and the like.

In addition, the emulsion composition for preventing or improving snoring of the present invention can also be ingested by addition to an existing food.

As the existing food to which the emulsion composition of the present invention can be added, drinks such as tea drinks, fruit juice beverage, coffee, beverage water and the like; liquid food such as soup, miso soup and the like; milk food such as cow milk, lactobacillus drinks and the like; semi-solid food such as jelly, yogurt, cream and the like; powder food such as instant soup, instant miso soup, instant coffee and the like, and the like are preferable.

The emulsion composition for preventing or improving snoring of the present invention is added to the above-mentioned food such that the daily ingestion amount falls within the above-mentioned range.

Furthermore, the present invention provides a method of preventing or improving snoring, comprising giving for ingestion or using the emulsion composition for preventing or improving snoring of the present invention in an amount effective for prevention or improvement of snoring to a subject in need of prevention or improvement of snoring.

The "subject in need of prevention or improvement of snoring" refers to those suffering from the symptoms of snoring and having a health or social or interpersonal hindrance due to various causes mentioned above.

While the amount of ingestion or use of the emulsion composition of the present invention can be appropriately determined depending on the age, sex, body weight of the subject, level of snoring and the like, it is preferable to ingest or use 50 mg - 600 mg, more preferably 100 mg - 300 mg, as the (A) vegetable oil, or the total amount of (A) vegetable oil and (D) plant essential oil of the emulsion composition of the present invention per day for an adult. The aforementioned amount of the emulsion composition of the present invention may be ingested or used as one dose or in several divided portions.

The method of preventing or improving snoring of the present invention uses an emulsion composition superior in emulsion stability, and therefore, can be applied conveniently and safely for a long term to a subject without requiring a homogenizing operation at the time of application.

In addition, the method of preventing or improving snoring of the present invention can well prevent or improve snoring of a subject.

### [Examples]

The present invention is explained in more detail in the following Examples.

### [Example 1] Emulsion composition for preventing or improving snoring

According to the formulation shown in Table 1, a liquid emulsion composition for preventing or improving snoring was prepared. First, (1) - (3) in Table 1 were mixed, and the homogenized oil phase component was heated to 80°C. On the other hand, (4) - (6) were added to (7) and dissolved, and the homogenized aqueous phase component was heated to 80°C. To the aforementioned aqueous phase component was added the aforementioned oil phase component with stirring, and the mixture was uniformly mixed and emulsified. The stirring was continued for about 2 min to give a homogeneous emulsion. The emulsion was cooled, components (8), (9) were successively added at 40°C, and the mixture was mixed and dissolved to be homogeneous. Then, (10) was added to adjust the pH of the emulsion composition to 3.9 - 4.6 and used as the emulsion composition for preventing or improving snoring of Example 1.

**Table 1**

| Component | | Content (wt%) |
|---|---|---|
| (1) | olive oil | 8.0 |
| (2) | sunflower oil | 2.0 |
| (3) | citric acid monoglyceride | 2.2 |
| (4) | xylitol | 27.5 |
| (5) | glycerol | 12.2 |
| (6) | preservative | 0.1 |
| (7) | purified water | amount making the total amount 100.0 |
| (8) | peppermint oil | 1.00 |
| (9) | vitamin B₆, C, E mixture | 0.6 |
| (10) | citric acid hydrate | q.s. |

### [Experimental Example 1] Evaluation of emulsion stability

The emulsion composition for preventing or improving snoring of Comparative Example 1 was prepared similarly to Example 1 except that xylitol was replaced with purified water in the emulsion composition for preventing or improving snoring of Example 1. The emulsion compositions of Example 1 and Comparative Example 1 were evaluated for the emulsion stability as shown below.

That is, the emulsion compositions of Example 1 and Comparative Example 1 were each placed by about 20 mL in a test tube with a stopper, sufficiently shaken after setting the stopper and allowed to stand at room temperature, and the emulsion state of the compositions was visually observed.

The emulsion stability of the emulsion compositions was evaluated according to the following evaluation criteria and shown in Table 2.

### <evaluation criteria of emulsion stability>

O; phase separation such as water separation, creaming and the like is not seen
Δ; phase separation such as water separation, creaming and the like is slightly seen
×; phase separation such as water separation, creaming and the like is remarkably seen

The state of each emulsion composition after 4 weeks is shown in Fig. 1.

**Table 2**

| Time lapsed | Sample | |
|---|---|---|
| | Example 1 | Comparative Example 1 |
| 1 week | ○ | x |
| 2 weeks | ○ | x |
| 3 weeks | ○ | x |
| 4 weeks | ○ | x |

As shown in Table 2 and Fig. 1, the emulsion composition of Example 1 of the present invention showed good emulsion stability, and did not show phase separation even after 4 weeks from preparation. On the other hand, the emulsion composition of Comparative Example 1 free of xylitol showed remarkable phase separation after a lapse of one week from preparation.

### [Example 2] Spray for pharynx

The emulsion composition for preventing or improving snoring of Example 1 was filled by 30 mL in a spray container with a metered-dose ejection mechanism (ejection amount per time=0.5 mL) and treated for sterilization. A pump dispenser was set to give the spray for pharynx of Example 2.

### [Industrial Applicability]

As mentioned above, according to the present invention, an emulsion composition for preventing or improving snoring, which is superior in emulsion stability and well suppresses separation and the like of the contained components such as oily component and the like during preservation, can be provided.

The emulsion composition for preventing or improving snoring of the present invention can be applied conveniently to a pharyngeal region without requiring a homogenizing operation at the time of application and without causing a foreign body sensation or an uncomfortable feeling, is effective for preventing or improving snoring, is hypoallergenic and highly safe, and can be ingested or used continuously.

This application is based on a patent application No. 2015-154194 filed in Japan, the contents of which are incorporated in full herein.

## Claims

1. An emulsion composition for preventing or improving snoring, comprising (A) a vegetable oil, (B) a sugar alcohol having a carbon number of not less than 4 and (C) an emulsifier.

2. The emulsion composition according to claim 1, further comprising (D) a plant essential oil.

3. The emulsion composition according to claim 1 or 2, wherein the (A) vegetable oil is one kind or two or more kinds selected from the group consisting of canola oil, olive oil, sunflower oil and safflower oil.

4. The emulsion composition according to any one of claims 1 to 3, wherein the (B) sugar alcohol having a carbon number of not less than 4 is one kind or two or more kinds selected from alditols having a carbon number of 5 - 6.

5. The emulsion composition according to any one of claims 1 to 4, wherein the (C) emulsifier is one kind or two or more kinds selected from the group consisting of glycerin fatty acid ester, sorbitan fatty acid ester and organic acid monoglyceride.

6. The emulsion composition according to any one of claims 2 to 5, wherein the (D) plant essential oil is an essential oil obtained from one kind or two or more kinds of plants of the genus Mentha.

7. The emulsion composition according to any one of claims 1 to 6, which is a liquid.

8. The emulsion composition according to any one of claims 1 to 7, which is sprayed on the pharynges.

9. An agent for preventing or improving snoring, comprising the emulsion composition according to any one of claims 1 to 6.

10. The agent according to claim 9, which is an oral liquid.

11. The agent according to claim 9, which is a spray for pharynges.

12. A food for preventing or improving snoring, comprising the emulsion composition according to any one of claims 1 to 6.

13. The food according to claim 12, which is a liquid.

14. The food according to claim 12, having a form of a spray.

15. The food according to any one of claims 12 to 14, which is a food with health claims, special purpose food or health supplement.
